# EUROPEAN PATENT APPLICATION

(11) **EP 2 369 549 A1**
(43) Date of publication of application: **28.09.2011**
(21) Application number: 10001939.7
(22) Date of filing: 25.02.2010
(51) Int. Cl.: G06Q 50/00

(54) **Method of performing a series of experiments, an integrated continuous pharmaceutical product system, and a computer program product**

(71) Applicant: GEA Pharma Systems NV, 2160 Wommelgem (BE)
(72) Inventor: Page, Trevor Gordon, Southampton, SO45 4RA (GB)
(74) Representative: Carlsson, Eva

(57) **Abstract**

In the method, a series of experiments is performed in order to explore a set of process parameter ranges and/or a set of ingredient ranges of a pharmaceutical processing system. The method comprises step 180, in which a set of process parameter ranges and/or a set of ingredient ranges is selected. In step 182 the series of experiments is run automatically in an integrated continuous pharmaceutical product processing system, in which the set of process parameter ranges and/or said set of ingredient ranges are explored. Measurement data is collected during the experiments taking into account the process dynamics in step 184. In step 186, a set of quality attributes is evaluated relative to at least one target attribute and/or quality attribute of a predetermined set of target attributes and/or quality attributes of a product, respectively. Finally, in step 188, the measurement data is stored. There may be an additional step 190 of adjusting at least one range of said set of process parameter ranges and/or said set of ingredient ranges during the series of experiments in response to differences between said set of target attributes and/or said set of quality attributes and the predetermined set of target attributes and/or quality attributes, respectively.

## Description

### Field of the invention

The present invention relates to a method of performing a series of experiments in order to explore a set of process parameter ranges and/or a set of ingredient ranges of a pharmaceutical product processing system. The invention furthermore relates to an integrated continuous pharmaceutical product processing system, and to a computer program product.

### Background of the invention

Within the pharmaceutical industry there is an increasing interest in providing products of a higher quality, Streamlining of a process line by including probes or sensors capable of in-line or on-line analysis may provide increased product quality and process efficiency, by making it possible to obtain analytical results before and after each step allowing each unit operation to be controlled on the basis of these results. In addition, still stricter requirements to process reproducibility and safety are to be expected from authorities granting marketing authorisations. Recent ideas behind how pharmaceutical processes should be designed and performed have been formulated as a set of guidelines by the Food and Drug Administration (FDA) in the USA. The FDA uses the term "Process Analytical Technology" (PAT), and in their Guidance for Industry regarding PAT (dated September 2004), it is stated that "the Agency considers PAT to be a system for designing, analysing, and controlling manufacturing through timely measurements (i.e., during processing) of critical quality and performance attributes of raw and in-process materials and processes, with the goal of ensuring final product quality. It is important to note that the term analytical in PAT is viewed broadly to include chemical, physical, microbiological, mathematical, and risk analysis conducted in an integrated manner. The goal of PAT is to enhance understanding and control the manufacturing process, which is consistent with our current drug quality system: quality cannot be tested into products; it should be built-in or should be by design. Consequently, the tools and principles described in this guidance should be used for gaining process understanding and can also be used to meet the regulatory requirements for validating and controlling the manufacturing process." An example of a virtual platform to facilitate automated batch production is given in US2005/0137735, wherein a plan for handling information streams and applying the information in a process design is suggested.

Typical manufacturing processes employed within the pharmaceutical field until now are of a batch nature. Batch manufacturing processes have a number of advantages and provide satisfactory results within many areas. However, due the increasingly widespread application of PAT criteria for monitoring and controlling in particular pharmaceutical manufacturing processes, and to the general increase in the demands to quality by design, the level of quality of monitoring and control attainable by a batch process is often not sufficient, i.a. due to the fact that settings are fixed. Furthermore, a relatively large buffer volume is required, entailing undesired back-mixing of the material stream and a limited traceability of the manufactured product. As a consequence, manufacturers' and customers' focus of interest has shifted to continuous processes, in which settings may be varied and are allowed to change within a design space. In order to achieve more production output with a batch process, bigger equipment and bigger buffer volumes, with different process settings to attain the same output, would be required. This is known as the scale-up problem. More output with a continuous process just requires longer running, with the ability to maintain the same settings. Further advantages of the continuous process include the ability to provide real-time release and its inherent advantages: Less product in stock, less quality testing, faster time-to-market, less costs involved etc. Furthermore, there is an increased interest for more robust processing equipment and for the ability to control more incoming variation, while maintaining tablet quality.

Ideally, the output from the processing machine corresponds to the aggregated input of ingredients at the feed or inlet points, i.e. all of the material is fed to the tabletting machine in a continuous flow and at a constant rate. Due to a variety of factors, this is not feasible in practice. It is under any circumstances almost impossible to adjust the output from the mixing and drying units to provide a just-in-time supply of material to the tabletting machine. In turn, this inevitably necessitates the use of intermediate buffer vessels in order to store material upstream of the tablet press.

Due to the many process parameters that influence the end product, and the mixing relationship between the ingredients the procedure of defining the correct settings is time-consuming. Each analysis is often performed on separate devices, and an operator would, based on his experience, have to estimate the right setting of the process parameters and the right mix of ingredients for each test run. First it would take time to carry out the experiments because there are so many possibilities to combine the many process parameter ranges with the various ingredient ranges, and subsequently it would take time to evaluate which parameter resulted in which result and was it this parameter and not another parameter that resulted in this result. Furthermore, as the processing parameters can only be changed as fast as the whole process can respond and reach steady state, a certain delay is unavoidable. A way to limit the time it takes to carry out the experiments is to limit the process parameter ranges to the area where you expect a good result. This leaves a great amount of ground untested.

Hence, in order to fully understand the process it is necessary to carry out a relatively large series of time-consuming experiments.

### summary of the invention,

On this background, it is an object of the present invention to provide a system that explores the process design space and stores the knowledge obtained during the series of experiments.

In a first aspect, these and further objects are met by a method of performing a series of experiments in order to explore a set of process parameter ranges and/or a set of ingredient ranges of a pharmaceutical product processing system, the method comprising:
selecting a set of process parameter ranges and/or;
selecting a set of ingredient ranges;
running the series of experiments automatically in an integrated continuous pharmaceutical product processing system, in which the set of process parameter ranges and/or said set of ingredient ranges are explored;
collecting measurement data during the experiments taking into account the process dynamics;
evaluating a set of quality attributes relative to at least one target attribute and/or quality attribute of a predetermined set of target attributes and/or quality attributes of a product, respectively; and
storing the measurement data.

By storing the measurement data from a series of experiments carried out in an integrated continuous pharmaceutical product processing system, the overall time required for performing the series of experiments may be reduced to a substantial extent in comparison with prior art methods. This is, inter alia, due to the fact that a substantially larger number of experiments may be carried out during a certain time period. In comparison with carrying out experiments in a traditional batch process plant, as much as five or six times more experiments per time unit may be carried out. In the integrated continuous system, the process is performed continuously, and the process parameter ranges and/or ingredient ranges are varied continuously. Some process parameters and some ingredient ranges may be left unchanged throughout the series of experiments. By "series of experiments" is meant that an array of experiments is carried out successively, and there is no clear division, for instance in terms of a batch that separates one experiment from another.

The selection of process parameter ranges and/or ingredient ranges may be performed manually, semi-automatically or automatically based on for instance previous series of experiments.

In the wording "integrated pharmaceutical product processing system", the term "integrated" means that the outlet of one unit of processing equipment feeds the next unit of processing equipment, by means of any form of conveying means, or is fed to an outlet in the form of a discharge station.

What is meant by "continuous" is that a least one unit of processing equipment along the processing line, comprising several units of processing equipment, is substantially continuously receiving an input of material and/or is continuously delivering an output material during the processing.

"Collecting measurement data" means that throughout the processing system at least one measurement device is located. The one or more measurement devices may be used to collect data regarding the process parameters, e.g. temperature, rotational speed or another process parameter that may or may not affect the final product. The measurement data may also comprise data collected about the product. By product is meant any ingredient, including active ingredients, excipients, support ingredients or any composition added to, taking part in or being the result of any process performed in the pharmaceutical processing system, from raw material to finished product fed to the processing unit. The word "material" may also be used for the product.

As this is a continuous process the process dynamics has to be considered in order to know which piece of product has been subjected to which kind of processing and under what conditions. The process dynamics comprises the nature of the product flow in the system, the static and dynamic behavior of processes and the residence time distribution of the product in the system.

A predetermined set of quality attributes may be the preferred set of quality attributes, but may also any other set of predetermined sets of quality attributes.

A target attribute is a process parameter setting that may define a preferred process parameter setting that e.g. lowers production costs, provides a faster production, generates a cheaper product than other settings, but may also provide a product with the predetermined set of quality attributes. Hence the process parameter settings may be taken into account in order to achieve both a target attribute and a quality attribute.

The evaluation may take place solely on the measured processing parameters, but it may also take place based on measurements on the product.

In a preferred embodiment, the method further comprises the step of:
adjusting at least one range of said set of process parameter ranges and/or said set of ingredient ranges during the series of experiments in response to differences between said set of target attributes and/or said set of quality attributes and the predetermined set of target attributes and/or quality attributes, respectively.

By adjusting the system correspondingly, the foundation for a good product design and process is formed. A quality grade may be introduced, basing the production on previous production data, which thus reduces time spent on testing even further. The experiment data may be from the R&D stage, from early development or other trials, or even from the manufacture of a specific product itself. The difference forming the basis of the adjusting step may be established manually or automatically. For instance, if two experiments result in the same product quality, one method might generate less maintenance, use less power, take shorter time etc. depending of which production factors the company prioritizes. This may be reflected in the target attribute. Hence, the system can be set up to determine the preferred set of process parameters or ingredient ranges without interference from operators or other involved persons. It may also be possible for the operator to select or set the process parameters to a certain range or value. The adjustment may also take place before the series of experiments are initiated or between individual experiments.

Preferably, the method may comprise the further step of logging measurement data, wherein the measurement data is selected from a group consisting of process parameters, ingredient information and measurements performed on the product during the experiments.

The step of determining the process dynamics may be carried out by means of at least one traceable ingredient. The traceable ingredient may be traceable by means of the amount of ingredient added, the colour of the ingredient, the composition of the ingredient and/or the humidity of the ingredient.

In principle, the at least one process parameter range may be any suitable, but is preferably selected from a group consisting of: temperature, humidity, pressure, energy input from granulation or blending, compression force, compression profile, rotational speed, production rate, pump speed, air flow, product flow rate, relative flow rate, tablet press setting or any combination thereof.

Correspondingly, the set of quality attributes of the product may be any characteristic or property of a product, and is preferably selected from a group consisting of: Composition data, moisture data, particle size distribution, weight, hardness, dissolution, shape, identity, disintegration, blend homogeneity, particle size distribution of a raw material, blend homogeneity of a mixing step, particle size distribution after granulation, content uniformity of the drug substance, droplet size distribution of coating solution, coating thickness or any combination thereof.

The term "temperature" could be any temperature measured throughout the process, such as temperature of the raw material, blending temperature, pressing temperature or coating temperature or any other temperature measurable throughout a production system.

The same applies to humidity, including temperature of the raw material, blending humidity, pressing humidity or coating humidity or any other humidity measurable throughout a production system. The pressure or the air pressure throughout the production at the various steps.

In a further aspect, an automated integrated continuous pharmaceutical product processing system for performing a series of experiments in order to explore a set of process parameter ranges is provided. The system comprises:
traceability means for determining a process dynamics of a product;
measurement means for measuring at least one process parameter of said process parameter ranges and/or at least one ingredient of said set of said set of ingredient ranges and/or at least one target attribute and/or at least one quality attribute during the series of experiments, thereby generating measurement data; and
a database for storing said measurement data.

In a still further aspect, a computer program product is provided, the computer program product comprising computer program code able to carry out any one of the steps according to any one of claims 1-7.

Further details and advantages appear from the dependent claims, and from the detailed description of preferred embodiments and examples for carrying out the method set forth below.

### Brief description of the drawings

Fg. 1 shows a flow diagram of the method according to the invention;
Fig. 2 shows a flow diagram in a preferred embodiment of the method according to the Invention;
Figs 3 to 5 each show a schematic overview of a system according to the invention, each Figure representing a different embodiment;
Fig. 6 shows a system of an embodiment of the invention.

### Detailed description of the invention and of preferred embodiments

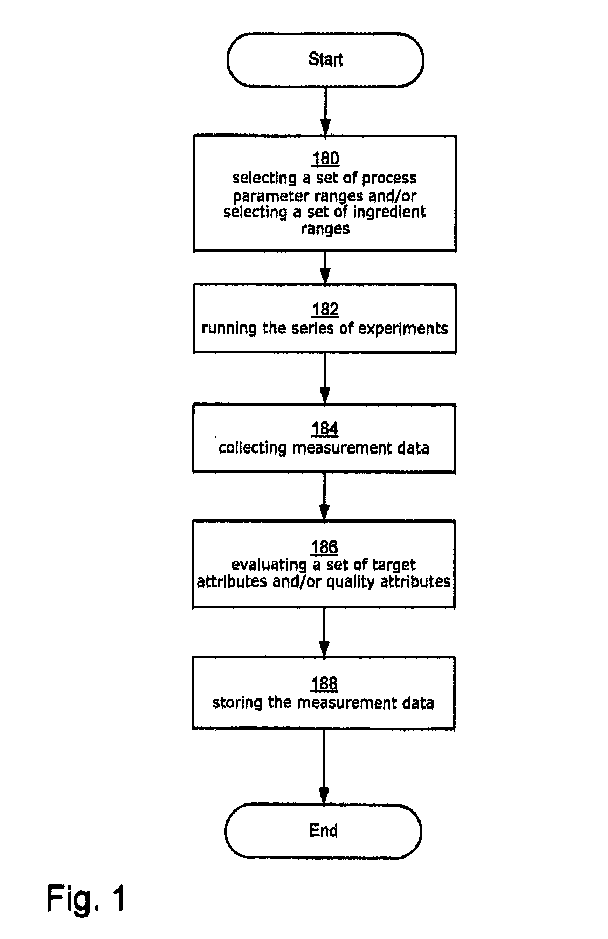

Referring now to the Figures, Fig. 1 shows a flow diagram representing the steps of the method according to the invention.

Details regarding the individual steps will be described in further detail below. In the method according to the invention, a series of experiments is performed in order to explore a set of process parameter ranges and/or a set of ingredient ranges of a pharmaceutical processing system. The method comprises step 180, in which a set of process parameter ranges and/or a set of ingredient ranges is selected. In step 182 the series of experiments is run automatically in an integrated continuous pharmaceutical product processing system, in which the set of process parameter ranges and/or said set of ingredient ranges are explored. Measurement data is collected during the experiments taking into account the process dynamics in step 184. In step 186, a set of quality attributes is evaluated relative to at least one target attribute and/or quality attribute of a predetermined set of target attributes and/or quality attributes of a product, respectively. Finally, in step 188, the measurement data is stored.

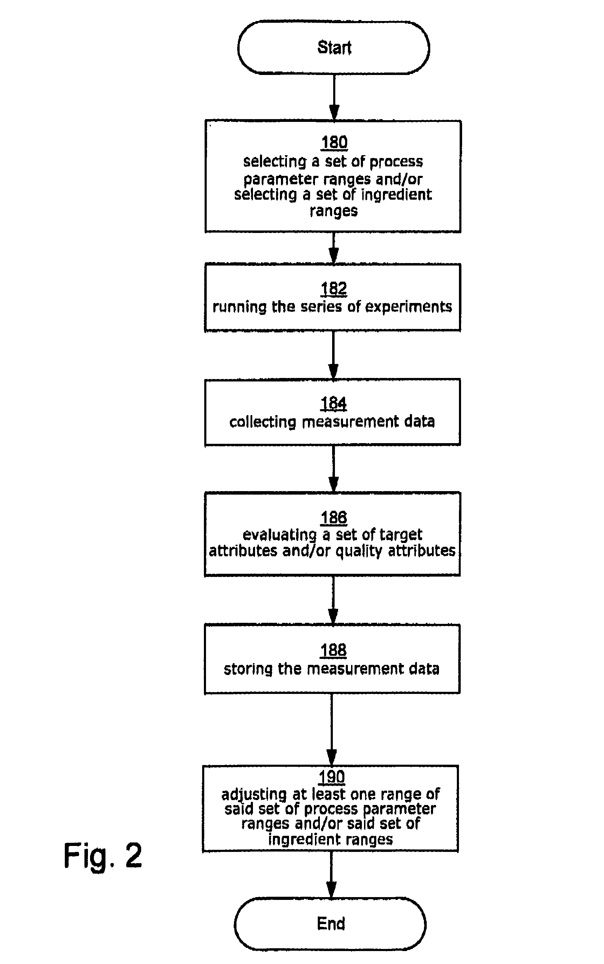

In Fig. 2, there is an additional step 190 of adjusting at least one range of said set of process parameter ranges and/or said set of Ingredient ranges during the series of experiments in response to differences between said set of target attributes and/or said set of quality attributes and the predetermined set of target attributes and/or quality attributes, respectively.

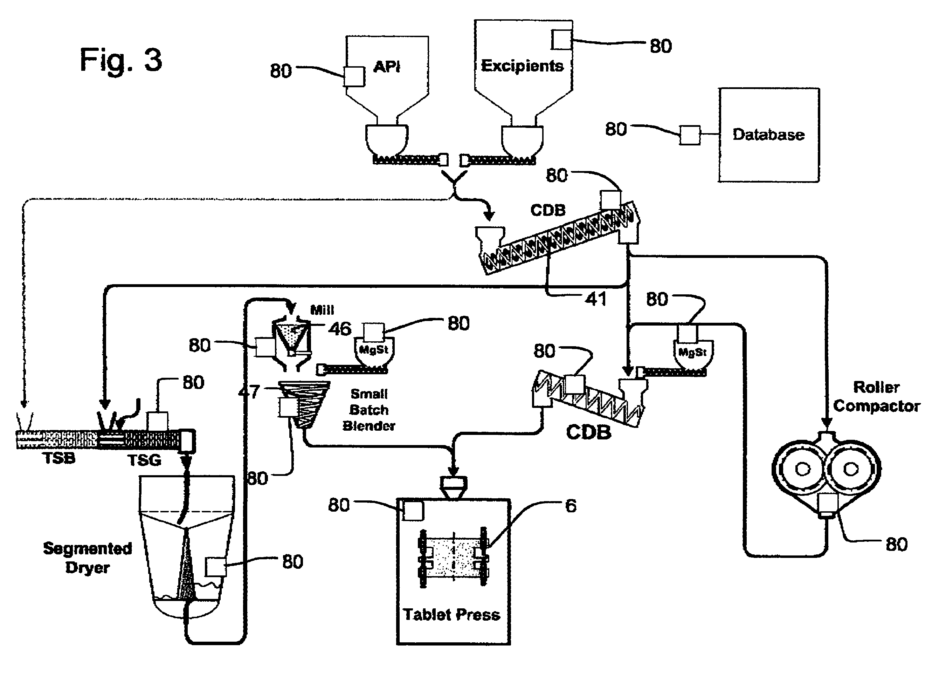

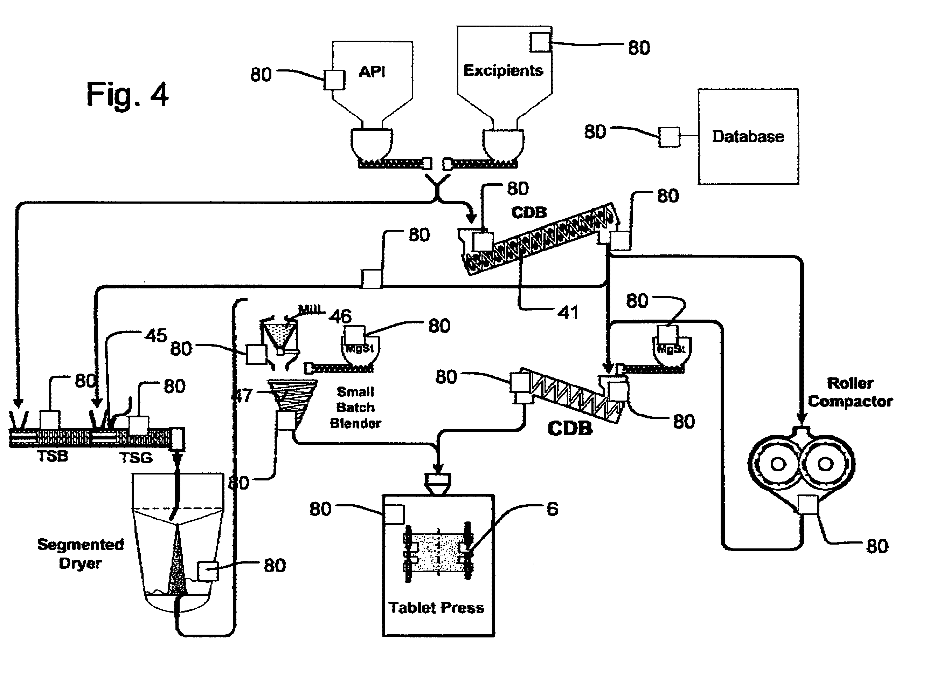

Referring now to Figs 3 to 5, examples of conceivable test, pilot or production plants in which the method for performing the series of experiments according to the invention may be applied will be described. In the examples, the plant is formed as a contained module as described in Applicant's co-pending international application, PCT/IB2009/051885, the contents of which are incorporated herein by reference. The plant or module comprises various processing devices or units, such as a blender, a mixer, a tablet press etc. Only units denoted by reference numerals form part of the process line; other units remain in the individual Figures 3 to 5 for reasons of convenience.

In the overview of Fig. 3, the module is designed for two different excipients, so that an initial excipient may enter the module at the inlets to the module, together with the API, and is processed in the first mixing unit 41, which is in the form of a continuous dry blender (CDB). A further excipient, such as a lubricant, e.g. magnesium stearate (MgSt), may enter the module and is brought together with the material stream leaving the Initial mixing unit 41 into a further mixing unit 42, which may also be in the form of a continuous dry blender. The material stream may be analysed in any suitable manner and at any suitable positions as described in the above before entering the tablet press 6.

Instead of directing the material stream directly from the initial mixing unit 41 to the further mixing unit 42, the material stream of the process line may also be directed via a roller compactor 43 compacting the material before it enters the further mixing unit 42.

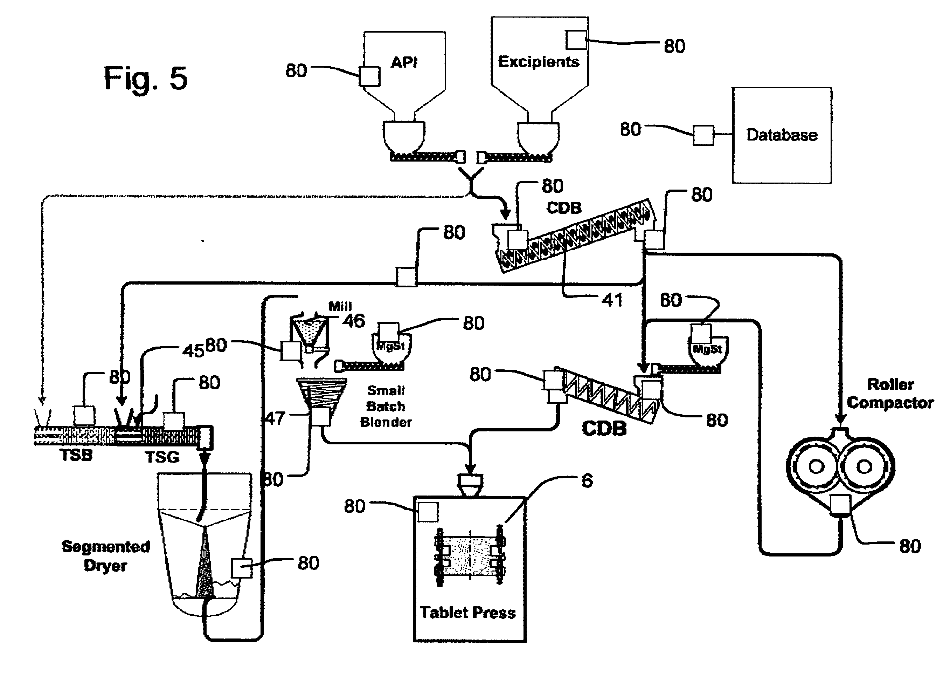

Alternatively, the mixing unit comprises a twin screw blender 44 (TSB) as indicated in Fig. 5, to which the material stream of API and excipients entering the module through the inlets is conducted. The twin screw blender 44 is followed by a twin screw granulator 45 (TSG) processing the material stream further. Following this operation, the material stream is guided to segmented dryer 5 and further to a mill 46. The further excipient, such as a lubricant, e.g. magnesium stearate, enters the module at this point and is brought together with the existing material stream, this mixture being blended in a small batch blender 47 before being fed to the tablet press 6.

As a further alternative, not shown, the material streams of API and excipients are brought together in the initial mixing unit 41, in the form of continuous dry blender, and then brought to the twin screw granulator 45 (thus not via the twin screw blender 44), and further through the dryer 5, mill 46, small batch blender 47 and then to tablet press 6.

On Figs. 3 to 5 measurement means 80 are positioned at various places throughout the test, pilot or production plant. The measurement means 80 may include any instrument suitable for measuring such as spectroscopic technique devices, Near Infrared devices, Transmittance devices, Near Infrared devices, diffuse reflectance near infrared spectroscopy (DRNIR) devices, Fourier transform infrared devices, laser light diffraction devices, mass spectrometer devices, hydrometers, weighing devices, thermometers, The device may be placed inside the individual processing devices such as at the inlet of the processing device, at the outlet of the processing device and/or outside the processing device. A Diffuse reflectance near infrared spectroscopy (DRNIR) device is suitable for determining component identity, moisture content, blend homogeneity, hardness, coating thickness, and/or material identity, transmittance NIR (T-NIR) is suitable for determining content uniformity and laser light diffraction (LLD) is suitable for determining particle size distribution.

The measurement data is then transferred to a database or another storage means.

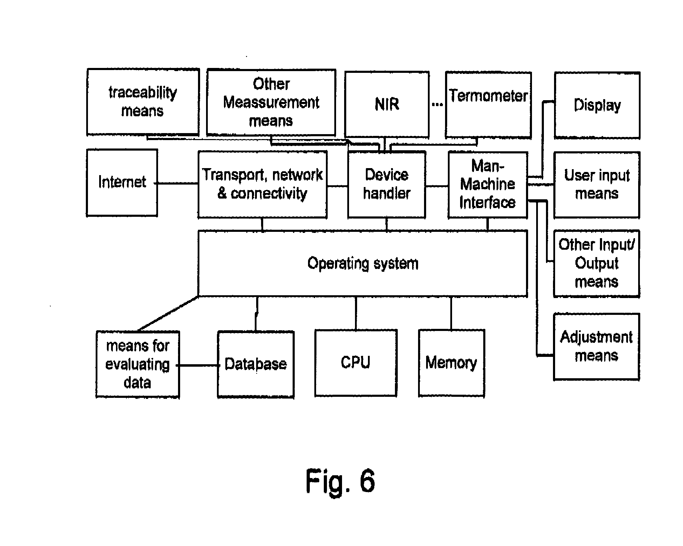

Fig 6 shows a schematic diagram over an automated integrated continuous pharmaceutical product processing system for performing a series of experiments in order to explore a set of process parameter ranges and a set of ingredient ranges. The test system may only comprise some of the shown elements and it may also comprise more or other elements. The elements may be connected in a different way than as shown here. The adjustment means is connected with the production system or pilot plant. The adjustment means is able to change the process parameter ranges.

In theory, an integrated continuous process may be run as a perfect plug flow, i.e. the material put into the inlet and at other locations in the process, is traceable throughout the process. Thus, in theory it would be possible to trace a certain piece of material or product from the inlet to the outlet. In combination with knowledge of the production speed and other factors, it would, theoretically, be possible to determine for instance the throughput time for a given piece of product. In practice, however, a certain amount of back-mixing occurs, thus rendering the possibility of tracing the material in the process more difficult. Hence, traceability means is provided for determining process dynamics, such as residence time distribution of a product. The system is adapted to receive at least one traceable ingredient.

Furthermore, measurement means is provided for measuring at least one process parameter of said process parameter ranges and/or at least one ingredient of said set of said set of ingredient ranges or a quality attribute during the series of experiments, thereby generating measurement data; and a database for storing said measurement data.

In preferred embodiments, means for evaluating the set of quality attributes measured during the series of experiments relative to a predetermined set of quality attributes of the product is provided.

Furthermore, adjustment means for adjusting at least one process parameter range of said set of process parameter ranges and/or at least one ingredient range of said set of ingredient ranges during the series of experiments. The adjustment means comprises means for adjusting at least one process parameter range of said set of process parameter ranges and/or at least one ingredient range of said set of ingredient ranges in response to differences between said measured set of quality attributes and the preferred set of quality attributes.

In the embodiment shown, the integrated continuous pharmaceutical product processing system is a tablet manufacturing system

Eventually, a computer program product is provided, comprising computer program code able to carry out any one of the steps of the method according to the invention.

The invention should not be regarded as being limited to the embodiments shown and described in the above. Several modifications and combinations are conceivable within the scope of the appended claims.

## Claims

1. Method of performing a series of experiments in order to explore a set of process parameter ranges and/or a set of ingredient ranges of a pharmaceutical processing system, the method comprising:
selecting a set of process parameter ranges and/or;
selecting a set of ingredient ranges;
running the series of experiments automatically in an integrated continuous pharmaceutical product processing system, in which the set of process parameter ranges and/or said set of ingredient ranges are explored;
collecting measurement data during the experiments taking into account the process dynamics;
evaluating a set of quality attributes relative to at least one target attribute and/or quality attribute of a predetermined set of target attributes and/or quality attributes of a product, respectively; and
storing the measurement data.

2. The method according to claim 1, further comprising the step of:
adjusting at least one range of said set of process parameter ranges and/or said set of ingredient ranges during the series of experiments in response to differences between said set of target attributes and/or said set of quality attributes and the predetermined set of target attributes and/or quality attributes, respectively.

3. The method according to any one of claims 1 or 2, further comprising the step of:
logging measurement data, wherein the measurement data is selected from a group consisting of process parameters, ingredient information and measurements performed on the product during the experiments.

4. The method according to any one of the preceding claims, wherein the step of determining the process dynamics is carried out by means of at least one traceable ingredient.

5. The method according to claim 4, wherein the traceable ingredient is traceable by means of the amount of ingredient added, the colour of the ingredient, the composition of the ingredient and/or the humidity of the ingredient.

6. The method according to any one of the preceding claims, wherein the at least one process parameter range is selected from a group consisting of: temperature, humidity, pressure, energy input from granulation or blending, compression force, compression profile, rotational speed, production rate, pump speed, air flow, product flow rate, relative flow rate, tablet press setting or any combination thereof.

7. The method according to any one of the preceding claims, wherein the set of quality attributes of the product is selected from a group consisting of: Composition data, moisture data, particle size distribution, weight, hardness, dissolution, shape, identity, disintegration, blend homogeneity, particle size distribution of a raw material, blend homogeneity of a mixing step, particle size distribution after granulation, content uniformity of the drug substance, droplet size distribution of coating solution, coating thickness or any combination thereof.

8. An automated integrated continuous pharmaceutical product processing system for performing a series of experiments in order to explore a set of process parameter ranges and a set of ingredient ranges, comprising:
traceability means for determining the process dynamics of a product;
measurement means for measuring at least one process parameter of said process parameter ranges and/or at least one ingredient of said set of said set of ingredient ranges and/or at least one target attribute and/or at least one quality attribute during the series of experiments, thereby generating measurement data; and
a database for storing said measurement data.

9. A system according to claim 8, wherein
means for evaluating the set of quality attributes measured during the series of experiments relative to a predetermined set of target attributes and/or quality attributes of the product; and
adjustment means for adjusting at least one process parameter range of said set of process parameter ranges and/or at least one ingredient range of said set of ingredient ranges during the series of experiments.

10. A system according to claim 9, wherein said adjustment means comprises means for adjusting at least one process parameter range of said set of process parameter ranges and/or at least one ingredient range of said set of ingredient ranges in response to differences between said measured set of target attributes and/or quality attributes and the preferred set of target attributes and/or quality attributes, respectively,

11. A system according to any one of claims 8 to 10, wherein the system is adapted to receive at least one traceable ingredient.

12. A system according to any one of claims 8 to 11, wherein the measurement means is selected from a group consisting of: spectroscopic technique devices, such as Near Infrared devices, Transmittance devices, Near Infrared devices, diffuse reflectance near infrared spectroscopy (DRNIR) devices, Fourier transform infrared devices, laser light diffraction devices, mass spectrometer devices, microwave devices, hydrometers, weighing devices, thermometers, soft sensors for sensing groups of parameters (modelling techniques), tablet analysing devices including means for measuring hardness, dissolution, disintegration and electromagnetic signature and an dielectric probe, THz measurement systems or any combination thereof.

13. A system according to any one of claims 8 to 12, wherein the integrated continuous pharmaceutical product processing system is a tablet manufacturing system

14. A computer program product comprising computer program code able to carry out any one of the steps according to any one of claims 1-7.

## Amended claims

### Amended claims in accordance with Rule 137(2) EPC.

**1.** Method of performing a series of experiments in order to explore a set of process parameter ranges and/or a set of ingredient ranges of a pharmaceutical processing system, the method comprising:
selecting a set of process parameter ranges and/or;
selecting a set of ingredient ranges;
running the series of experiments automatically in a pharmaceutical product processing system, in which the set of process parameter ranges and/or the set of ingredient ranges are explored;
evaluating a set of quality attributes relative to at least one target attribute and/or quality attribute of a predetermined set of target attributes and/or quality attributes of a product, respectively; and
storing measurement data, **characterized in that** the series of experiments are run in an integrated continuous pharmaceutical product processing system and **in that** the measurement data are collected during the experiments taking into account the process dynamics.

**2.** The method according to claim 1, further comprising the step of:
adjusting at least one range of said set of process parameter ranges and/or said set of ingredient ranges during the series of experiments in response to differences between said set of target attributes and/or said set of quality attributes and the predetermined set of target attributes and/or quality attributes, respectively.

**3.** The method according to any one of claims 1 or 2, further comprising the step of:
logging measurement data, wherein the measurement data is selected from a group consisting of process parameters, ingredient information and measurements performed on the product during the experiments.

**4.** The method according to any one of the preceding claims, wherein the step of determining the process dynamics is carried out by means of at least one traceable ingredient.

**5.** The method according to claim 4, wherein the traceable ingredient is traceable by means of the amount of ingredient added, the colour of the ingredient, the composition of the ingredient and/or the humidity of the ingredient.

**6.** The method according to any one of the preceding claims, wherein the at least one process parameter range is selected from a group consisting of: temperature, humidity, pressure, energy input from granulation or blending, compression force, compression profile, rotational speed, production rate, pump speed, air flow, product flow rate, relative flow rate, tablet press setting or any combination thereof.

**7.** The method according to any one of the preceding claims, wherein the set of quality attributes of the product is selected from a group consisting of: Composition data, moisture data, particle size distribution, weight, hardness, dissolution, shape, identity, disintegration, blend homogeneity, particle size distribution of a raw material, blend homogeneity of a mixing step, particle size distribution after granulation, content uniformity of the drug substance, droplet size distribution of coating solution, coating thickness or any combination thereof.

**8.** An automated pharmaceutical product processing system for performing a series of experiments in order to explore a set of process parameter ranges and a set of ingredient ranges, comprising:
measurement means for measuring at least one process parameter of said process parameter ranges and/or at least one ingredient of said set of said set of ingredient ranges and/or at least one target attribute and/or at least one quality attribute during the series of experiments, thereby generating measurement data; and
a database for storing said measurement data **characterized in that** the system is an integrated continuous pharmaceutical product processing system and **in that** the system comprises traceability means for determining the process dynamics of a product.

**9.** A system according to claim 8, wherein
means for evaluating the set of quality attributes measured during the series of experiments relative to a predetermined set of target attributes and/or quality attributes of the product; and
adjustment means for adjusting at least one process parameter range of said set of process parameter ranges and/or at least one ingredient range of said set of ingredient ranges during the series of experiments.

**10.** A system according to claim 9, wherein said adjustment means comprises means for adjusting at least one process parameter range of said set of process parameter ranges and/or at least one ingredient range of said set of ingredient ranges in response to differences between said measured set of target attributes and/or quality attributes and the preferred set of target attributes and/or quality attributes, respectively.

**11.** A system according to any one of claims 8 to 10, wherein the system is adapted to receive at least one traceable ingredient.

**12.** A system according to any one of claims 8 to 11, wherein the measurement means is selected from a group consisting of: spectroscopic technique devices, such as Near Infrared devices, Transmittance devices, Near Infrared devices, diffuse reflectance near infrared spectroscopy, DRNIR, devices, Fourier transform infrared devices, laser light diffraction devices, mass spectrometer devices, microwave devices, hydrometers, weighing devices, thermometers, soft sensors for sensing groups of parameters, modelling techniques, tablet analysing devices including means for measuring hardness, dissolution, disintegration and electromagnetic signature and an dielectric probe, THz measurement systems or any combination thereof.

**13.** A system according to any one of claims 8 to 12, wherein the integrated continuous pharmaceutical product processing system is a tablet manufacturing system

**14.** A computer program product comprising computer program code able to carry out any one of the steps according to any one of claims 1-7.
